# EUROPEAN PATENT APPLICATION

(11) **EP 2 243 775 A1**
(43) Date of publication of application: **27.10.2010**
(21) Application number: 09157523.3
(22) Date of filing: 07.04.2009
(51) Int. Cl.: C07D 307/79, C07D 307/80

(54) **Synthesis of 1-(2,3-Dihydrobenzofuran-4-YL)ethanone as intermediate in the preparation of ramelteon**

(71) Applicant: LEK Pharmaceuticals d.d., 1526 Ljubljana (SI)
(72) Inventor: The designation of the inventor has not yet been filed

(57) **Abstract**

The present invention relates in general to the field of organic chemistry and in particular to the preparation of 1-(2,3-dihydrobenzofuran-4-yl)ethanone, an intermediate in preparation of (S)-N-[2-(1,6,7,8-tetrahydro-2H-indeno-[5,4-b]furan-8-yl)ethyl]propionamide, i.e. ramelteon.

## Description

### Field of the Invention

The present invention relates in general to the field of organic chemistry and in particular to the preparation of 1-(2,3-dihydrobenzofuran-4-yl)ethanone, an intermediate in preparation of (S)-N-[2-(1,6,7,8-tetrahydro-2H-indeno-[5,4-b]furan-8-yl)ethyl]propionamide, *i.e.* ramelteon.

### Background of the Invention

Ramelteon, (S)-N-[2-(1,6,7,8-tetrahydro-2H-indeno-[5,4-b]furan-8-yl)ethyl]propion- amide, is a melatonin receptor agonist with both high affinity for melatonin MT1 and MT2 receptors and selectivity over the MT3 receptor. Ramelteon demonstrates full agonist activity in vitro in cells expressing human MT1 or MT2 receptors, and high selectivity for human MT1 and MT2 receptors compared to the MT3 receptor. The activity of ramelteon at the MT1 and MT2 receptors is believed to contribute to its sleep-promoting properties, as these receptors, acted upon by endogenous melatonin, are thought to be involved in the maintenance of the circadian rhythm underlying the normal sleep-wake cycle.

The synthesis of ramelteon is disclosed in EP885210B1, EP1792899A1 and J. Med Chem. 2002, 45, 4222-4239. Ramelteon is synthesized in two parts; first the synthesis of the tricyclic core with the key intermediate 6,7-dihydro-1H-indeno[5,4-b]furan-8(2H)-one is performed in six or seven steps and then the side chain with the introduction of the chirality and amide function is performed in four steps. The synthesis uses 2,3-benzofuran as starting material and in several steps involves the use of small to large excess of halogenated reagents.

International patent application W02008/106179 A1 discloses a ten step synthesis of ramelteon via alternative intermediates. The synthesis uses excess of halogenated reagents, as well as reagents such as liquid ammonia and borontribromide.

International patent application W02008/151170 A2 describes the preparation of ramelteon via key intermediate 6,7-dihydro-1H-indeno[5,4-b]furan-8(2H)-one which is in 6 steps transformed to ramelteon. Excess of halogenated reagents and reagents such as liquid ammonia and borontrifluoride are used.

### Summary of the invention

The present invention provides the following items including main aspects and preferred embodiments, which respectively alone and in combination particularly contribute to solving the above object and eventually provide additional advantages:
(1) A process for preparing a compound of formula V 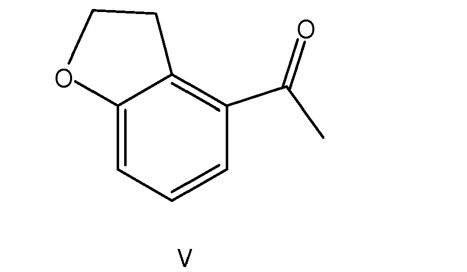 from a compound of formula III 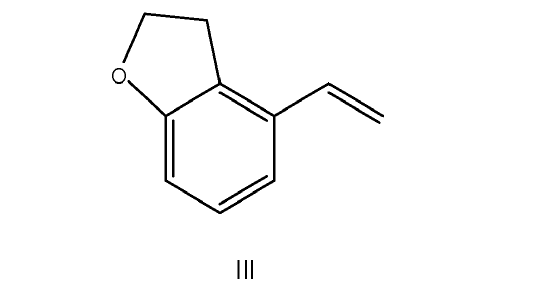 comprising a step of converting the vinyl group of the compound of formula III into ethanone group to give the compound of formula V.
(2) The process according to item (1) comprising the step of reacting a compound of formula III with an oxidant in the presence of catalyst.
(3) The process according to item (2) wherein said step of reacting a compound of formula III with an oxidant in the presence of catalyst is performed in the presence of ionic liquid.
(4) The process according to item (3), wherein the ionic liquid is selected from the compounds having general formulae IVa and IVb: 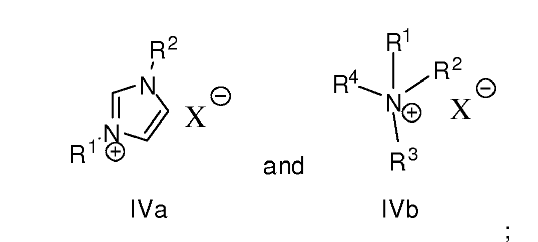 wherein R¹, R², R³ and R⁴ are each independently selected from the group consisting of independently substituted or unsubstituted alkyl, cycloalkyl, alkenyl, cycloalkenyl, alkynyl, aryl, arylalkyl, arylcycloalkyl,heteroaryl, arylalkyl and heteroarylalkyl group, and X is selected from the group consisting of Cl, Br, I, SO₄, NO₃, BF₄, PF₆, [(CF₃SO₂)₂N] and CF₃SO₃, most preferably ionic liquid is a compound of formula IVa, wherein R¹ is carboxymethyl, R² is 1,2-dicarboxyethyl and X is Cl, or a compound of formula IVa, wherein R¹ is n-Bu, R² is Me and X is BF₄ or PF₆.
(5) The process according to any of items (3) and (4), wherein said ionic liquid is used in an amount of less than 0.02 molar equivalents compared to compound III, more preferably the amount of ionic liquid used is less then 0.01 molar equivalents compared to compound of formula III, most preferably the amount of ionic liquid used is about 0.005 molar equivalents compared to compound of formula III.
(6) The process according to any one of items (2) - (5), wherein said catalyst is a metal catalyst selected from the group consisting of Pd, Au and Pt catalysts.
(7) The process according to item (6), wherein Pd catalyst is used, preferably said catalyst is PdCl₂.
(8) The process according to any one of items (2) - (7), wherein said oxidant is selected from oxygen and H₂O₂.
(9) The process according to item (8), wherein said oxidant is H₂O₂.
(10) The process according to any one of items (1) - (9), wherein said compound of formula III is prepared by a process comprising reacting a compound of formula II 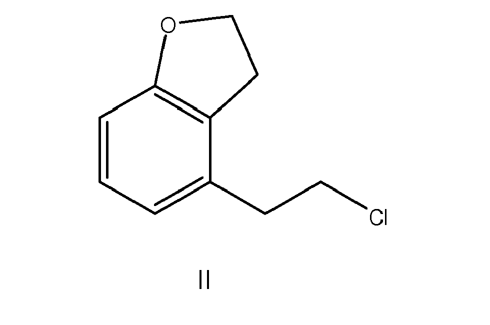 with base in biphasic media to give a compound of formula III.
(11) The process according to item (10), wherein said base is selected from the group of hydroxides, preferably the base is NaOH.
(12) The process according to any one of items (10) and (11), wherein said process for preparing the compound of formula III is performed in the presence of phase transfer agent.
(13) The process according to item (12), wherein said phase transfer agent is selected from the group consisting of tetraalkyl ammonium salts of general formula R⁵₄NX wherein R⁵ is selected from substituted and unsubstituted alkyl group and wherein X is selected from the group consisting of Cl, Br, I, SO₄ and OH, preferably said phase transfer agent is Bu₄NOH.
(14) A process for preparing a compound of formula II 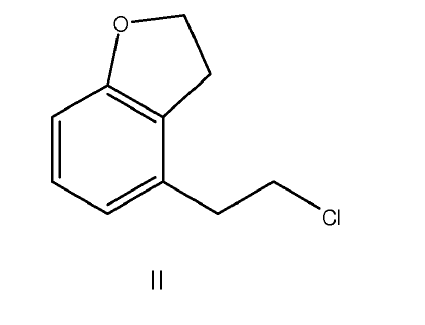 comprising the steps of:
   a) in situ preparation of Vilsmeier reagent by reacting oxalyl chloride with DMF,
   b) reacting compound of formula I with Vilsmeier reagent obtained from step a) to yield the compound of formula II 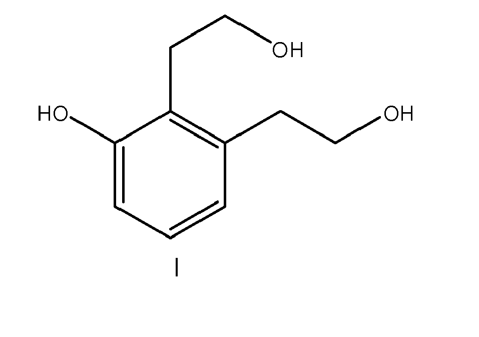
(15) The process according to item (14), wherein steps a) and b) are performed in one pot without isolation of Vilsmeier reagent obtained from step a).
(16) The process according to any one of items (14) and (15), wherein compound of formula I is added not before the Vilsmeier reagent is completely formed.
(17) The process according to any one of items (14) to (16), wherein step a) and b) is performed at a temperature below 0°C, preferably at a temperature from -30°C to -10°C, more preferably at a temperature from -22°C to -18°C.
(18) The process according to any one of items (10) - (13), wherein said compound of formula II is prepared by a process according to any one of items (14) to (17).
(19) A process for preparing the compound of formula V comprising the steps of:
   a) preparing the compound of formula II by a process comprising the steps of:
      - in situ preparation of Vilsmeier reagent from oxalyl chloride and DMF and
      - reacting compound of formula I with Vilsmeier reagent to yield a compound of formula II;
   b) reacting the compound of formula II with base selected from the group of hydroxides, wherein preferably the base is NaOH, in biphasic media, in the presence of phase transfer agent selected from the group consisting of tetraalkyl ammonium salts of general formula R⁵₄NX wherein R⁵ is selected from substituted and unsubstituted alkyl group and wherein X is selected from the group consisting of Cl, Br, I, SO₄ and OH, to yield a compound of formula III, wherein a preferable phase transfer agent is Bu₄NOH;
   c) reacting the compound of formula III with an oxidant selected from the group consisting of oxygen and H₂O₂ in the presence of catalyst, wherein said catalyst is a metal catalyst selected from the group consisting of Pd, Au and Pt catalysts, preferably the catalyst is PdCl₂, and in the presence of ionic liquid, wherein said ionic liquid is selected from the compounds having general formulae IVa and IVb: 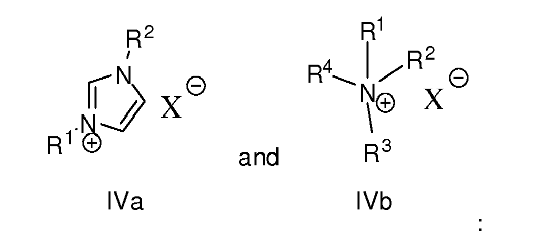 wherein R¹ , R², R³ and R⁴ are each independently selected from the group consisting of independently substituted or unsubstituted alkyl, cycloalkyl, alkenyl, cycloalkenyl, alkynyl, aryl, arylalkyl, arylcycloalkyl,heteroaryl, arylalkyl and heteroarylalkyl group, and X is selected from the group consisting of Cl, Br, I, SO₄, NO₃, BF₄, PF₆, [(CF₃SO₂)₂N] and CF₃SO₃. Most preferably ionic liquid is a compound of formula IVa, wherein R¹ is carboxymethyl, R² is 1,2-dicarboxyethyl and X is Cl, or a compound of formula IVa, wherein R¹ is n-Bu, R² is Me and X is BF₄ or PF₆. 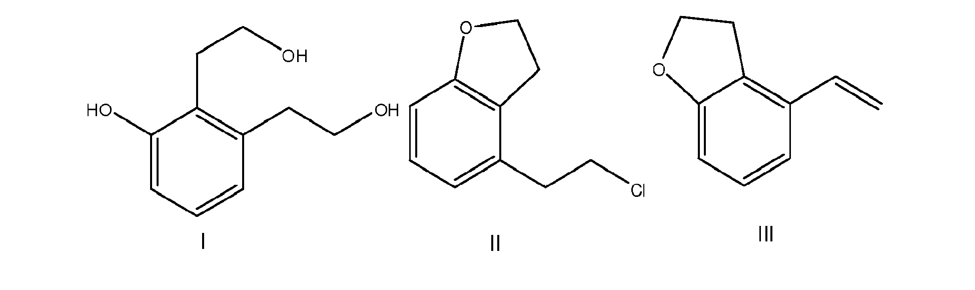 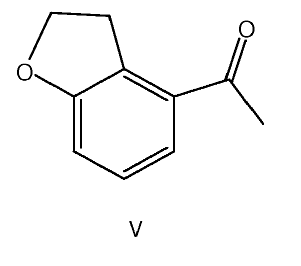
(20) The process according to any one of items (1)- (19), wherein the respectively specified process step, alone or in combination, is controlled using Process Analytical Technology (PAT) using real time detection of at least one of educts and products by Fourier transform infrared spectroscopy (FTIR).
(21) The process according to any one of items (1)-(13), (19) and (20), wherein said compound of formula V is further converted to compound of formula VI, said conversion comprising the steps of
   a) reacting a compound of formula V with paraformaldehyde in the presence of ammonium salt, R⁶R⁷NH₂⁺X⁻, (wherein R⁶ and R⁷ are each independently selected from alkyl, cycloalkyl, aryl, arylalkyl and arylcycloalkyl; and X is halogen, BF₄, PF₆, H₂PO₄ or R⁸CO₂, wherein R⁸ is one of alkyl, aryl, polyhaloalkyl) in organic solvent;
   b) contacting the solution from step a) with strong inorganic acid and obtaining compound of formula VI 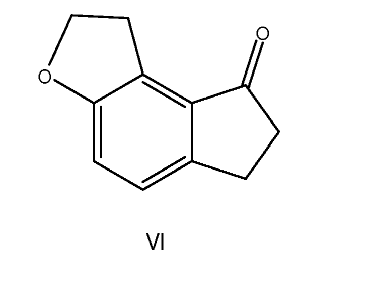
(22) A process for the preparation of ramelteon, comprising the steps of:
   carrying out a process for preparing the compound of formula V according to any one of items (1)-(13) and (19)-(20); and
   subjecting the compound of formula V to further synthesis steps to yield ramelteon.
(23) A process for the preparation of ramelteon, comprising the steps of:
   carrying out a process for preparing the compound of formula VI according to item (21); and
   subjecting the compound of formula VI to further synthesis steps to yield ramelteon.
(24) Use of a compound of formula III 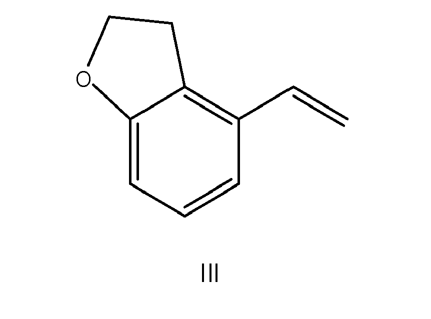 for the synthesis of ramelteon.
(25) A process for the preparation of a pharmaceutical composition comprising ramelteon as active ingredient, comprising the steps of:
   preparing ramelteon according to the process according to any one of the items (22) - (23) or according to the use of item (24), and
   admixing the thus prepared ramelteon with at least one pharmaceutically acceptable excipient.

The invention solves the problem of long and tedious synthesis of tricycle 6,7-dihydro-1H-indeno[5,4-b]furan-8(2H)-one, which is a useful intermediate for further synthesis, in particular for the synthesis of ramelteon. Embodiments of relevant process steps according to this invention proceed via 4-(2-chloroethyl)-2,3-dihydrobenzofuran, 4-vinyl-2,3-dihydrobenzofuran and 1-(2,3-dihydrobenzofuran-4-yl)ethanone, which respectively represent prior intermediates themselves being useful for synthetically providing the desired key intermediate of tricyclic 6,7-dihydro-1H-indeno[5,4-b]furan-8(2H)-one, and which altogether are short and efficient and provide yields that are industrially applicable and competitive. The procedural concept according to the present invention uses cheap starting materials, and its steps altogether involve only four steps to provide the desired key intermediate. Further, compared to prior art processes it is possible that reduced amounts of halogenated reagents are used, and toxic and/or hazardous reagents such as liquid ammonia, borontrifluoride and borontribromide are not needed. According to this invention said whole process and individual reaction steps are susceptible to applying process analytical technology (PAT) to individual reaction steps, which thereby enables optimization of reaction conditions (e.g. reagents amounts, reaction times and safety).

### Detailed description of the Invention

In the following, the present invention will be described in more detail by preferred embodiments and examples noting, however, that these embodiments, examples are presented for illustrative purposes only and shall not limit the invention in any way.

The term "about" generally means within 10%, preferably 5% and more preferably within 1 % of a given value or range. Alternatively, the term "about" means within an acceptable standard error of the mean, when considered by one of the ordinary skill in the art.

As used herein, the terms "alkyl", "cycloalkyl","aryl", "arylalkyl", "heteroaryl", and "heteroarylalkyl" may adopt their usual and known meanings. More specifically, "alkyl" means straight or branched alkyl of 1 to 10 carbon atoms, preferably 1 to 8 carbon atoms and more preferably 1 to 6 carbon atoms, "cycloalkyl" means cycloalkyls of 3 to 8 carbon atoms, "aryl" means substituted or unsubstituted aryls selected from a single six-membered ring or condensed six-membered rings, preferably phenyl or naphtyl, more preferably phenyl, "arylalkyl" means substituted or unsubstituted phenylalkyl, where alkyl is 1 to 6 carbon atoms, "heteroaryl" means aromatic rings of 5 to 7 carbon atoms where 1, 2 or 3 carbon atoms are exchanged by oxygen, nitrogen or sulphur, and "heteroarylalkyl" means the aforementioned heteroaryls comprising alkyl of 1 to 6 carbon atoms. Any aforementioned alkyl, aryl, arylalkyl or heteroarylalkyl can be optionally unsaturated in its alkyl moiety, or substituted in its aromatic and/or alkyl moiety with one or more substituents selected from alkyl of 1 to 4 carbon atoms, F, Cl, Br, OH, OCH₃, CF₃, and COOR⁹, where R⁹ is H, alkyl of 1 to 4 carbon atoms, phenyl, alkenyl or alkynyl of 2 to 10 carbon atoms.

Reaction Scheme 1 illustrates a preferred embodiment of the process according to the present invention for preparing 1-(2,3-dihydrobenzofuran-4-yl)ethanone (V), which is valuable as an important intermediate in preparation of ramelteon.

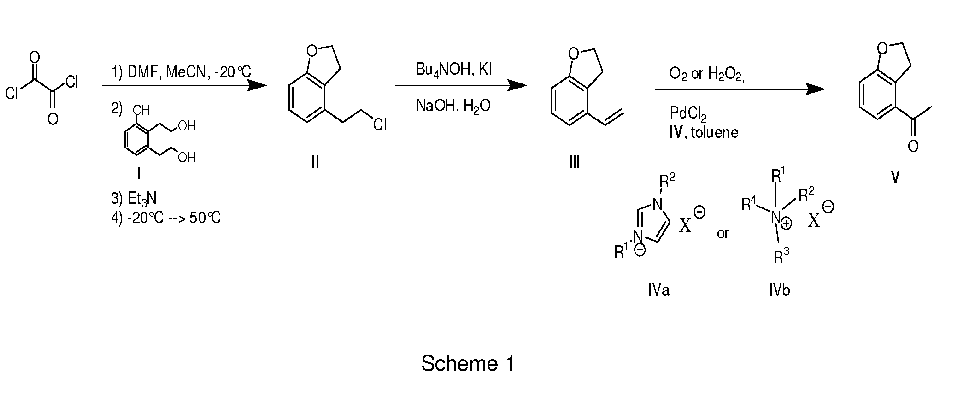

According to the preferred embodiment of Scheme 1, compound of formula II is prepared by a process comprising the steps of:
a) in situ preparation of Vilsmeier reagent (*i*. *e*. N-(chloromethylene)-N,N-dimethylammonium chloride) from oxalyl chloride and DMF
b) reacting compound of formula I with Vilsmeier reagent to yield a compound of formula II

Reaction is performed in organic solvent, preferably in MeCN. Said organic solvent is cooled, preferably at temperature from -30°C to -10°C, more preferably at temperature from -22°C to -18°C. To organic solvent oxalyl chloride and subsequently DMF is added, preferably under stirring, to form N-(chloromethylene)-N,N-dimethylammonium chloride, a so called "Vilsmeier reagent", in situ. This is a complex and multistep reaction involving short living Vilsmeier adducts followed by cyclization of one chain to dihydrofurane ring and chlorination of the other one. Such complex reactions may produce unwanted by-products e. g. in this case intermolecular couplings and intermediate compounds. Using isolated Vilsmeier reagent as it is described in literature gives some benefits in reaction control but an isolation of pure Vilsmeier reagent is a troublesome procedure or if commercially supplied more expensive option. It is advantageous to use simple chemicals like oxalyl chloride and DMF for industrial purposes but a complex reaction like this becomes uncontrollable because unconsumed oxalyl chloride may form additional side reaction. It has been found significant that the formation of the Vilsmeier reagent should efficiently be controlled during reaction by in-line control using PAT, preferably PAT-FTIR is used *(i.e*. In-line FTIR probe that measures characteristic FTIR bands of reagents, intermediates and products and allows continuous following of processes in real time). Such reaction control is even more desired if no special isolation of II is carried out and the product is transferred to the next steps without purification as all side product would also be transferred to the next step.

Thus when detecting that Vilsmeier reagent is completely formed and slight excess of DMF is observed, compound I is added portion wise, while the reaction mixture is kept at temperature below 0°C, preferably at temperature from -30°C to -10°C, more preferably at temperature from -22°C to -18°C. Formation of the intermediate is preferably controlled using PAT, in particular using PAT-FTIR. After the completion of formation of intermediate, tertiary amine, preferably Et₃N, is added drop wise, while the reaction mixture is still kept at temperature below 0°C, preferably at temperature from -30°C to -10°C, more preferably at temperature from -22°C to -18°C. Subsequently the reaction mixture is warmed up, preferably to a temperature above 40°C, more preferably to a temperature at about 50°C, preferably under stirring. Formation of compound of formula II is preferably controlled using PAT, in particular using PAT-FTIR. After reaction completion, the reaction mixture is cooled down to a temperature below 30°C, more preferably at around 20°C and quenched, preferably by adding water. Extractive work up furnishes compound of formula II in organic phase which is preferably stored at temperature below 10°C, preferably at temperature around 4°C. The step of synthesizing the compound of formula II therefore represents a process which is useful of its own and can be adavantageously used also for other purposes and synthesis schemes. In a preferred embodiment, this step is particularly adapted to the preparation of the compound of formula III as described in the following.

Specifically, further according to the preferred embodiment of Scheme 1, compound of formula III is prepared by a process comprising reacting a compound of formula II with base to give a compound of formula III, wherein said base is preferably selected from the group of hydroxides, most preferably base is NaOH. This reaction is carried out in biphasic media, composed of basic aqueous phase and organic phase. Preferably the combination of ether, preferably methyl tert-butyl ether (MTBE), and water is used.

Said process is preferably performed in the presence of phase transfer agent, preferably selected from the group consisting of tetraalkyl ammonium salts of general formula R⁵₄NX wherein R⁵ is selected from substituted and unsubstituted alkyl group and wherein X is selected from the group consisting of Cl, Br, I, SO₄ and OH, most preferably phase transfer agent is Bu₄NOH. Reaction is preferably performed in presence of catalytic amount of iodide anion to accelerate the reaction. Preferably less than 0.2 molar equivalents of iodide ion compared to compound of formula II is used, more preferably about 0.1 molar equivalents of iodide ion compared to compound of formula II is used. Source of iodide anion can be selected from the group consisting of compounds represented by the general compounds of formulae R⁵₄NI and MI, wherein R⁵ is as defined above and wherein M is selected from the group consisting of alkaline and alkaline earth metals, preferably iodide anion source is KI. Reaction is preferably performed under stirring at a temperature above 30°C, more preferably at temperature at about 50°C. Reaction is preferably followed using PAT, in particular using PAT-FTIR.

Extractive work up furnishes compound of formula III in organic phase, preferably ether and especially MTBE which is preferably stored at temperature below 10°C, preferably at temperature around 4°C until next step.

As such, the compound of formula III is useful as an intermediate for the synthesis of ramelteon.

Further according to the preferred embodiment of Scheme 1 the compound of formula III is advantageously used to prepare the compound of formula V using a Wacker reaction process. The Wacker reaction is a conversion of a vinyl group to an acetyl one in the presence of metal catalysts, especially transition metal catalysts. In certain cases, especially in the case of styrenes which are very succeptible to polymerization, yields might not be industrially acceptable. Up to date the reaction has been reported mostly only on simple styrenes and not on more complicated systems, and especially not for a compound of formula III.

Use of non-solvent conditions in the presence of catalytic amount of ionic liquids, as known from the literature, would appear to pose a problem in the present case because the product of formula V is a solid and the reaction mixture therefore would become impossible to stir.

It has been found that the oxidation of the compound of formula III in the presence of metal catalyst such as PdCl₂ can be effectively carried out in presence of ionic liquid to give compound V. Ionic liquid could be present in a large amount, such as more than 4 molar equivalents of respective ionic liquid substance compared to compound of formula III. However using large amounts of ionic liquid is economically disadvantageous. Surprisingly the reaction can as well be performed using concentrated solvent solution and minimum amounts of ionic liquid, while being carefully controlled by PAT.

The compound of formula III is therefore reacted with an oxidant in the presence of catalyst, preferably metal catalyst and especially a transition metal catalyst. Said step of reacting a compound of formula III with an oxidant in the presence of catalyst is preferably performed in the presence of ionic liquid.

Ionic liquid herein represents a compound that is in liquid form and completely in ionic state at about room temperature. It acts as co-catalyst and helps to enhance the reaction rate.

Preferably ionic liquid is selected from the compounds having a general formulae: 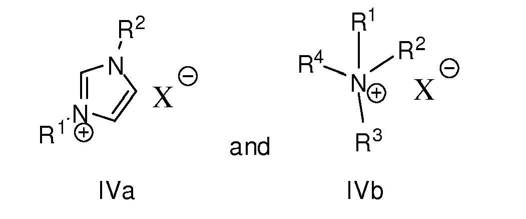 wherein R¹, R², R³ and R⁴ are each independently selected from the group consisting of independently substituted or unsubstituted alkyl, cycloalkyl, alkenyl, cycloalkenyl, alkynyl, aryl, arylalkyl, arylcycloalkyl,heteroaryl, arylalkyl and heteroarylalkyl group, and X is selected from the group consisting of Cl, Br, I, SO₄, NO₃, BF₄, PF₆, [(CF₃SO₂)₂N] and CF₃SO₃. Most preferably ionic liquid is a compound of formula IVa, wherein R¹ is carboxymethyl, R² is 1,2-dicarboxyethyl and X is Cl, or a compound of formula IVa, wherein R¹ is n-Bu, R² is Me and X is BF₄ or PF₆.

Preferably ionic liquid is used in the amount of less than 0.02 molar equivalents compared to compound III, more preferably the amount of ionic liquid used is less then 0.01 molar equivalents compared to compound of formula III, and most preferably the amount of ionic liquid used is about 0.005 molar equivalents compared to compound of formula III.

Said metal catalyst is preferably selected from the group consisting of Pd, Au and Pt catalysts, preferably Pd catalyst is used and most preferably catalyst is PdCl₂, Optionally said catalyst is used in combination with stochiometric amount of copper salts such as CuCl or CuCl₂, although preferably no copper salt is used.

Said oxidant is selected from the group consisting of oxygen and H₂O₂, preferably oxidant is H₂O₂, more preferably from about 1 to about 1.5 molar equivalents of H₂O₂ compared to compound of formula III is used.

The reaction is preferably performed in concentrated apolar solvent solution selected from the group consisting of aromatics, alkanes or halogenated solvents. Preferably the reaction is performed in toluene (preferably 0.5 to 6 weight equivalents of toluene compared to compound of formula III is used). The reaction mixture should be kept at temperature from 0 to 100°C, preferably at a temperature from 40 to 70°C, more preferably at about 55°C. Formation of the intermediate is preferably controlled using PAT, most preferably using PAT-FTIR. Extractive work up furnishes compound of formula V. Optionally purification, preferably by flash chromatography, is performed to yield pure compound of formula V.

The intermediate compound of formula V, 1-(2,3-dihydrobenzofuran-4-yl)ethanone (V), can then be subjected to further synthesis steps to yield ramelteon, preferably through intermediate 6,7-dihydro-1H-indeno[5,4-b]furan-8(2H)-one (VI).

Though other synthetic routes are feasible, according to the preferred embodiment of this invention the intermediate of formula VI is prepared according to a process illustrated in reaction scheme 2.

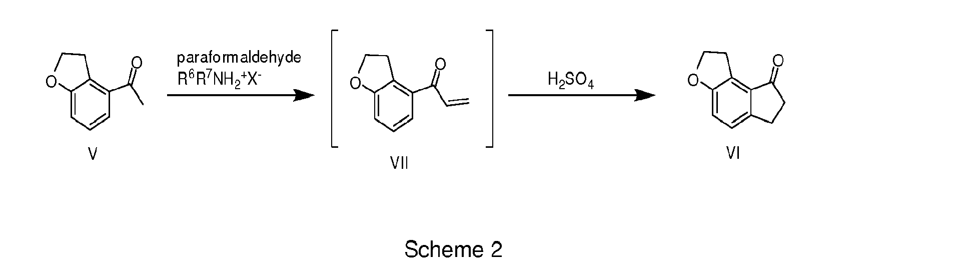

According to the preferred embodiment of Scheme 2, a compound of formula V is reacted with paraformaldehyde in the presence of an ammonium salt of formula R⁶R⁷NH₂⁺X⁻, (wherein R⁶ and R⁷ are each independently selected from alkyl, cycloalkyl, aryl, arylalkyl and arylcycloalkyl; and X is halogen, BF₄, PF₆, H₂PO₄ or R⁸CO₂, wherein R⁸ is one of alkyl, aryl, polyhaloalkyl), such as for example TADCA (dicyclohexylammonium 2,2,2-trifluoroacetate), TAMT (N-methyltoluidinium 2,2,2-trifluoroacetate) or TAMA (N-methylanilinium 2,2,2-trifluoroacetate) or TAMT (N-methyltoluidinium 2,2,2-trifluoroacetate).

An excess of the ammonium salt (up to 1 equivalent) can be used.

The reaction is preferably performed in aprotic solvent for 1 to 36 hours, more preferably for 2-12 hours, at about 60 to 120^{º}C.

At this stage acrylate intermediate VII can be effectively obtained in the form of a solution in organic solvent. The organic solvent is suitably an apolar solvent and is preferably selected from the group of alkanes, ethers or chlorinated solvents. Advantageously, it is not necessary that intermediate VII is isolated but is subjected in solution to further reaction.

The solution is then reacted with strong inorganic acid, preferably sulfuric acid, at a temperature between 0 to 100°C, preferably 30°C to 70^{º}C to give a compound of formula VI.

The intermediate compound of formula VI, 6,7-dihydro-1H-indeno[5,4-b]furan-8(2H)-one, can then be subjected to further synthesis steps to yield ramelteon by synthesis route known to or readily devisable by a person skilled in the art, suitably involving the introduction of the side chain having chirality and amide function. The documents mentioned infra are incorporated herein by way of reference. For example, the following synthesis route may be applied:

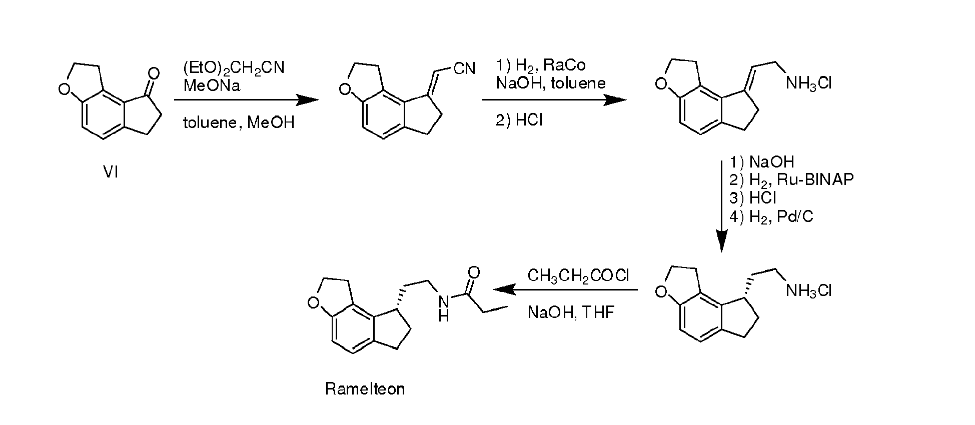

For preparing a pharmaceutical composition comprising ramelteon as active ingredient, first ramelteon is provided by the process as described above, and then the thus prepared ramelteon is admixed with at least one suitable pharmaceutically acceptable excipient. Pharmaceutically acceptable excipients may be selected from the group consisting of binders, diluents, disintegrating agents, stabilizing agents, preservatives, lubricants, fragrances, flavoring agents, sweeteners and other excipients known in the field of the pharmaceutical technology. Preferably, carriers and excipients may be selected from the group consisting of lactose, microcrystalline cellulose, cellulose derivatives, e.g. hydroxypropylcellulose, polyacrylates, calcium carbonate, starch, colloidal silicone dioxide, sodium starch glycolate, talc, magnesium stearate, polyvinylpyrrolidone, polyethylene glycol and other excipients known in the field of the pharmaceutical technology.

### Experimental Procedures

### Example 1:

### Preparation of 4-(2-chloroethyl)-2,3-dihydrobenzofuran (II)

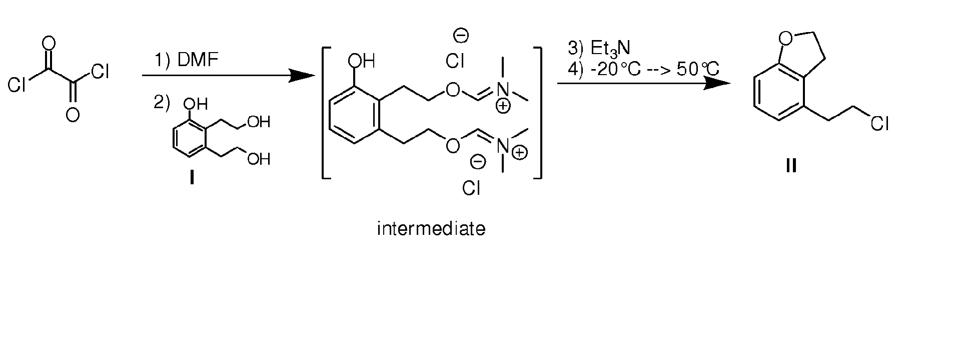

FTIR spectra of MeCN (140 ml) was recorded as reference. MeCN was cooled to -20°C, oxalyl chloride (16.5 ml) was added at once and waited until temperature re-stabilized at -20°C. DMF (16.6 ml) was then added drop-wise (temperature between -18°C and -22°C, 0.5 ml/min). Reaction was stirred until no oxalyl chloride was visible and DMF level was stable by FTIR. Vilsmeier reagent is thereby formed in situ according to the following reaction:

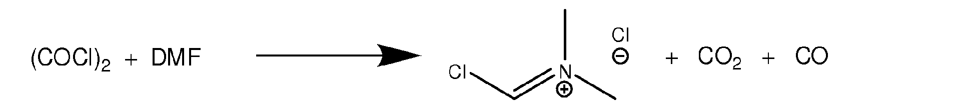

Product I was then added portion wise (temperature between -18°C and -21 °C, about 30 min). Formation of intermediate was immediately observed by FTIR. Reaction was stirred for one hour. Et₃N was then added drop-wise (temperature between -18°C and -22°C, 50 ml/h). At the end of addition, reaction was stirred 15 min at -20°C and temperature was slowly raised to 50°C (within about 15 min). Disappearance of intermediate and formation of DMF and product II was monitored by FTIR. When reaction looked completed by FTIR (about 2h at 50°C), the reaction was cooled down to 20°C and quenched with water (45 ml). Solution was transferred to a round bottom flask and MeCN was removed under reduced pressure. Solution was then diluted with MTBE (100 ml) and water (50 ml). Phases were separated and aqueous phase was re-extracted twice with MTBE (50 ml). Combined organic phases were washed twice with 10% H₃PO₄/10% NaCl solution and stored at 4°C until next step.

List of FTIR bands used to follow the reaction (using 2^{nd} derivative and solvent subtraction): Oxalyl chloride (reactant): Height to two point baseline, peak from 1800 cm⁻¹ to 1770 cm⁻¹, baseline 1800 cm⁻¹ to 1770 cm⁻¹.

Intermediate: Height to single point baseline, peak from 1722 cm⁻¹ to 1712 cm⁻¹, baseline 1722 cm⁻¹.

Compound II (product): Area to two point baseline, peak from 993 cm⁻¹ to 981 cm⁻¹, baseline 993 cm⁻¹ to 981 cm⁻¹.

DMF: Height to single point baseline, peak from 1694 cm⁻¹ to 1680 cm⁻¹, baseline 1694 cm⁻¹.

### Example 2:

### Preparation of 4-vinyl-2,3-dihydrobenzofuran (III)

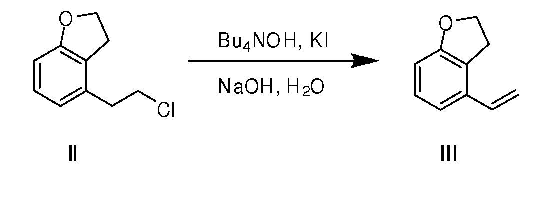

FTIR spectra of MTBE was recorded prior to the reaction as reference. To the solution of 4-(2-chloroethyl)-2,3-dihydrobenzofuran (II) in MTBE (150 ml) obtained at the previous step, was added, water (38 ml), KI (1.37 g), Bu₄NOH 40% (19 ml) and NaOH 50% solution (66 ml). Reaction was vigorously stirred and heated at 50°C until reaction looked completed by FTIR (4 to 5 h). Warm reaction mixture was then transferred into an extraction funnel to give three phases. Water phase (bottom) was removed and did not contain product. Medium phase (colored black) was diluted with water (120 ml) and was extracted three times with MTBE. Combined organic phases were washed twice with water, once with 0.5M NaHSO₃/10% NaCl solution and once with 1N NaOH/10% NaCl solution. MTBE solution was dried using MgSO₄, filtered, concentrated and used immediately for next step.

List of FTIR bands used to follow the reaction (using 2^{nd} derivative and solvent subtraction)
Compound II (reactant): Area to zero, peak from 1440 cm⁻¹ to 1437 cm⁻¹
Compound III (product): Area to zero, peak from 1417 cm⁻¹ to 1412 cm⁻¹.
Compound III (product): Area to zero, peak from 1565 cm⁻¹ to 1562 cm⁻¹.

### Example 3:

### Preparation of 1-(2,3-dihydrobenzofuran-4-yl)ethanone (V)

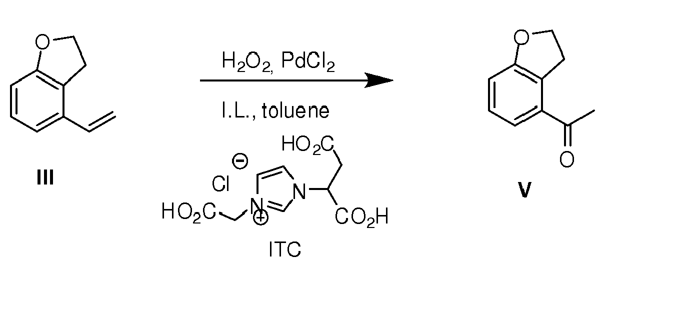

4-vinyl-2,3-dihydrobenzofuran (III) (2.4 g) was dissolved in toluene (2 ml) and were successively added (ITC) (51 mg), PdCl₂ (30 mg) and H₂O₂ 30% (2 ml). Reaction was vigorously stirred at 55°C until reaction looked completed by FTIR. (for around 24 h). Reaction was cooled down to room temperature, diluted with EtOAc (50 ml) and water (50 ml). Phases were separated and organic phase was washed with 0.5M NaHSO₃/10% NaCl solution and twice with 1 M NaHCO₃, dried over MgSO₄ and concentrated. Purification by flash chromatography gave 1-(2,3-dihydrobenzofuran-4-yl)ethanone (V). ¹H NMR □ (CDCl₃) 7.35 (dd, 1H, J = 0.8 Hz, J = 7.8 Hz), 7.19 (t, 1H, J = 7.9 Hz), 6.95 (d, 1H, J = 8.0 Hz), 4.57 (t, 2H, J = 8.8 Hz), 3.52 (t, 2H, J = 8.8 Hz), 2.57 (s, 3H). ¹³C NMR □ (CDCl₃) 198.8, 161.0, 133.8, 128.2, 127.9, 121.4, 113.4, 71.6, 31.0, 27.6.

List of FTIR bands used to follow the reaction (using 2^{nd} derivative and solvent subtraction) Compound III (reactant): Area to single point baseline, peak from 925 cm⁻¹ to 915 cm⁻¹, baseline 915 cm⁻¹.

Compound V (product): Area to zero, peak from 1730 cm⁻¹ to 1724 cm⁻¹.

### Example 4:

### Preparation of 6,7-dihydro-1H-indeno[5,4-b]furan-8(2H)-one (VI)

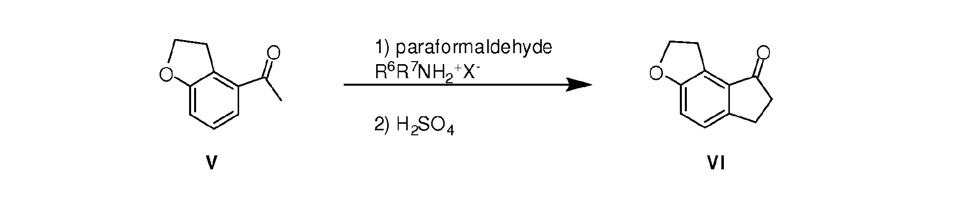

1-(2,3-dihydrobenzofuran-4-yl)ethanone (V) (1 g, 6.2 mmol) was dissolved in dioxane (9 ml). TADCA (dicyclohexylammonium 2,2,2-trifluoroacetate) (1.82 g, 1 eq) and paraformaldehyde (0.611 g, 1.1 eq) were added. The reaction was heated at 100°C for 2 h. A second portion of TADCA (0.91 g, 0.5 eq) and paraformaldehyde (0.333 g, 0.6 eq) were added and the reaction was heated at 100°C for 2 h. Reaction was partitioned between water (20 ml) and pentane (30 ml). Aqueous phase was re-extracted 4 times with pentane (10 ml). Combined pentane phases were washed with water and brine, dried over MgSO₄. Solution was diluted to 100 ml with pentane. This solution was added dropwise to a pre-heated solution of sulfuric acid at 67°C (10 ml) under nitrogen stream. At the end of addition, the reaction was stirred for 30 min. Reaction was cooled down to room temperature and poured on iced water (50 ml). Solution was extracted 5 times with MTBE. Combined organic phases were washed with water, NaHCO₃ 1 M and brine, dried over MgSO₄ and concentrated. Purification by flash chromatography furnished pure 6,7-dihydro-1H-indeno[5,4-b]furan-8(2H)-one (VI). ¹H NMR □ (CDCl₃) 7.21 (dd, 1H, J = 0.9 Hz, J = 9.0 Hz), 7.02 (d, 1H, J = 8.2 Hz), 4.66 (t, 2H, J = 8.9 Hz), 3.48 (t, 2H, J = 8.9 Hz), 3.08 (dd, 2H, J = 4.9 Hz, J = 6.0 Hz), 2.69 (m, 2H). ¹³C NMR □ (CDCl₃) 207.5, 160.2, 147.1, 133.6, 125.6, 123.9, 115.6, 72.3, 37.1, 28.4, 25.4.

## Claims

1. A process for preparing a compound of formula V 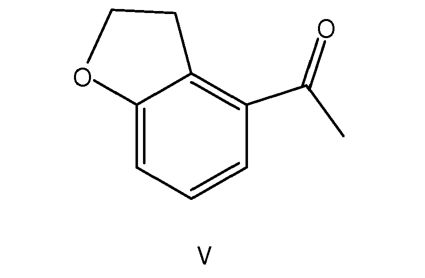 from a compound of formula III 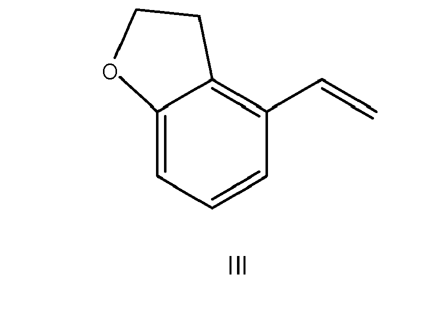 comprising a step of converting the vinyl group of the compound of formula III into ethanone group to give the compound of formula V.

2. The process according to claim 1, comprising the step of reacting a compound of formula III with an oxidant in the presence of catalyst, preferably said catalyst is a metal catalyst selected from the group consisting of Pd, Au and Pd catalysts, more preferably Pd catalyst is used, most preferably said catalyst is PdCl₂.

3. The process according to claim 2, wherein said step of reacting a compound of formula III with an oxidant in the presence of catalyst is performed in the presence of ionic liquid.

4. The process according to claim 3, wherein the ionic liquid is selected from compounds having general formulae IVa and IVb: 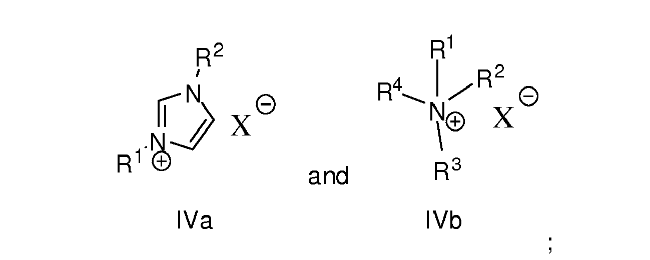 wherein R¹ , R², R³ and R⁴ are each independently selected from the group consisting of independently substituted or unsubstituted alkyl, cycloalkyl, alkenyl, cycloalkenyl, alkynyl, aryl, arylalkyl, arylcycloalkyl,heteroaryl, arylalkyl and heteroarylalkyl group, and X is selected from the group consisting of Cl, Br, I, SO₄, NO₃, BF₄, PF₆, [(CF₃SO₂)₂N] and CF₃SO₃, most preferably ionic liquid is a compound of formula IVa, wherein R¹ is carboxymethyl, R² is 1,2-dicarboxyethyl and X is Cl, or a compound of formula IVa, wherein R¹ is n-Bu, R² is Me and X is BF₄ or PF₆.

5. The process according to any one of claims 2-4, wherein said oxidant is selected from oxygen and H₂O₂.

6. The process according to any one of claims 2-5, wherein said compound of
formula III has been prepared by reacting a compound of formula II 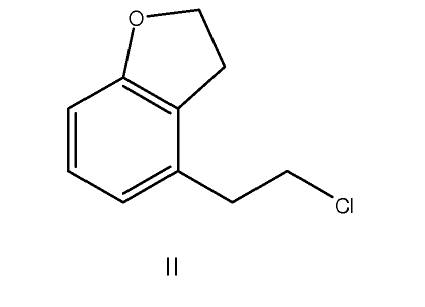 with base in biphasic media to give a compound of formula III.

7. The process according to claim 6, wherein said base is selected from the group of hydroxides, preferably the base is NaOH.

8. The process according to any one of claims 6 and 7, wherein said process for preparing the compound of formula III is performed in the presence of phase transfer agent, preferably said phase transfer agent is selected from the group consisting of tetraalkyl ammonium salts of general formula R⁵₄NX wherein R⁵ is selected from substituted and unsubstituted alkyl group and wherein X is selected from the group consisting of Cl, Br, I, SO₄ and OH, preferably said phase transfer agent is Bu₄NOH.

9. A process for preparing a compound of formula II 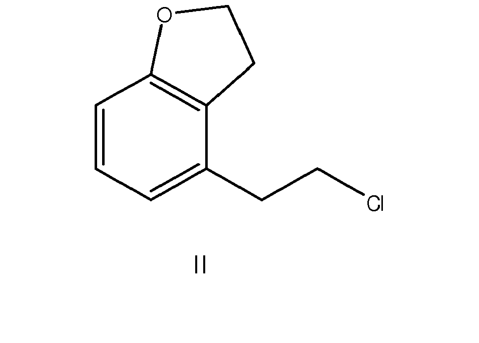 comprising the steps of:
a) in situ preparation of Vilsmeier reagent by reacting oxalyl chloride with DMF,
b) reacting compound of formula I with Vilsmeier reagent obtained from step a)
to yield the compound of formula II

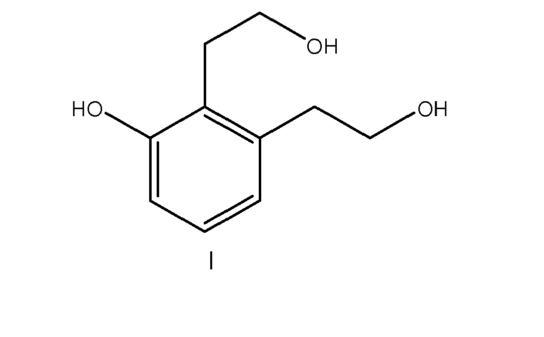

10. A process for preparing the compound of formula V comprising the steps of:
a) preparing the compound of formula II by a process comprising the steps of:
- in situ preparation of Vilsmeier reagent from oxalyl chloride and DMF and
- reacting compound of formula I with Vilsmeier reagent to yield a compound of formula II;
b) reacting the compound of formula II with base selected from the group of hydroxides, wherein preferably the base is NaOH, in biphasic media, in the presence of phase transfer agent selected from the group consisting of tetraalkyl ammonium salts of general formula R⁵₄NX wherein R⁵ is selected from substituted and unsubstituted alkyl group and wherein X is selected from the group consisting of Cl, Br, I, SO₄ and OH, to yield a compound of formula III, wherein a preferable phase transfer agent is Bu₄NOH; and
c) reacting the compound of formula III with an oxidant selected from the group consisting of oxygen and H₂O₂ in the presence of catalyst, wherein said catalyst is a metal catalyst selected from the group consisting of Pd, Au and Pt catalysts, preferably the catalyst is PdCl₂, and in the presence of ionic liquid, wherein said ionic liquid is selected from compounds having general formulae IVa and IVb: 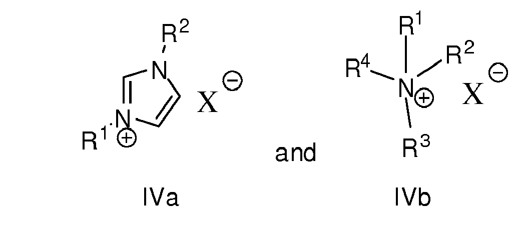 wherein R¹ , R², R³ and R⁴ are each independently selected from the group consisting of independently substituted or unsubstituted alkyl, cycloalkyl, alkenyl, cycloalkenyl, alkynyl, aryl, arylalkyl, arylcycloalkyl,heteroaryl, arylalkyl and heteroarylalkyl group, and X is selected from the group consisting of Cl, Br, I, SO₄, NO₃, BF₄, PF₆, [(CF₃SO₂)₂N] and CF₃SO₃, most preferably ionic liquid is a compound of formula IVa, wherein R¹ is carboxymethyl, R² is 1,2-dicarboxyethyl and X is CI, or a compound of formula IVa, wherein R¹ is n-Bu, R² is Me and X is BF₄ or PF₆.

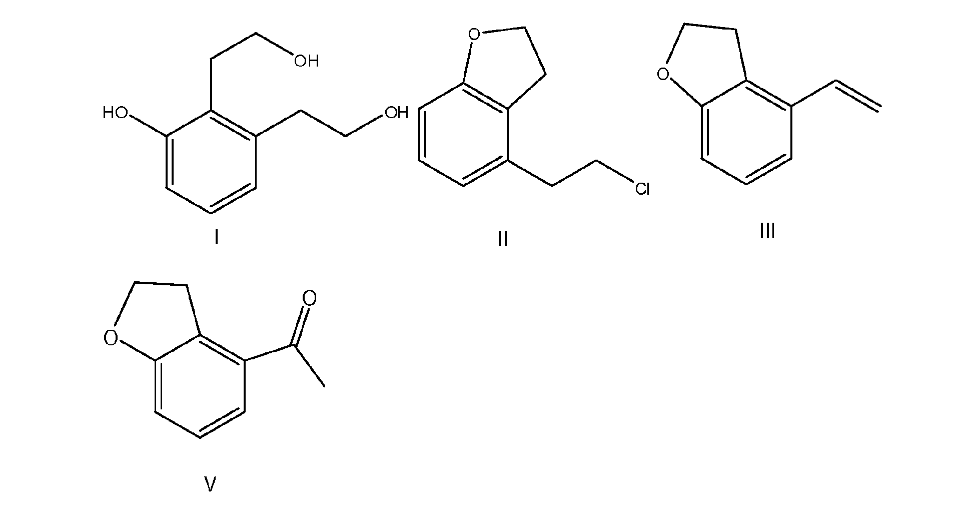

11. The process according to any one of claims 1-10, wherein the respectively specified process step, alone or in combination, is controlled using Process Analytical Technology (PAT) using real time detection of at least one of educts and products by Fourier transform infrared (FTIR) spectroscopy (FTIR).

12. A process for the preparation of ramelteon, comprising the steps of carrying out a process for preparing the compound of formula V according to any one of claims 1-8, 10 and 11; and subjecting the compound of formula V to further synthesis steps to yield ramelteon.

13. The process for the preparation of ramelteon according to claim 12, wherein the further synthesis steps to yield ramelteon proceeds via a compound of formula VI, prepared from the compound of formula V comprising the steps of
a) reacting a compound of formula V with paraformaldehyde in the presence of ammonium salt, R⁶R⁷NH₂⁺X⁻, (wherein R⁶ and R⁷ are each independently selected from alkyl, cycloalkyl, aryl, arylalkyl and arylcycloalkyl; and X is halogen, BF₄, PF₆, H₂PO₄ or R⁸CO₂, wherein R⁸ is one of alkyl, aryl, polyhaloalkyl) in organic solvent;
b) contacting the solution from step a) with strong inorganic acid and obtaining compound of formula VI

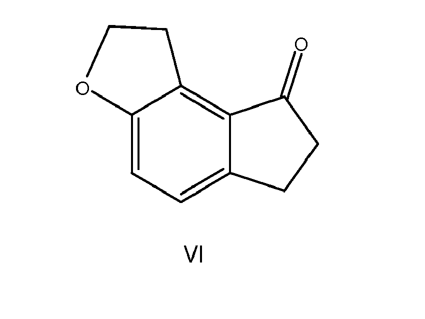

14. Use of a compound of formula III 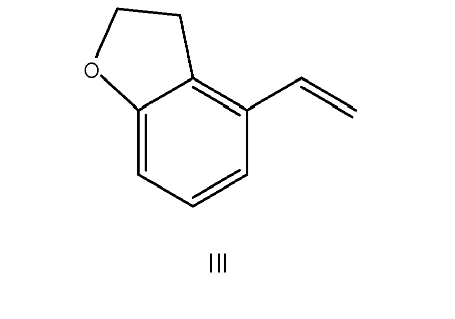 for the synthesis of ramelteon.

15. A process for the preparation of a pharmaceutical composition comprising ramelteon as active ingredient, comprising the steps of:
preparing ramelteon according to the process according to any one of the claims 12-13 or according to the use of claim 14, and
admixing the thus prepared ramelteon with at least one pharmaceutically acceptable excipient.
